(19) Europäisches Patentamt
European Patent Office
Office européen des brevets

(11) **EP 4 810 011 A1**

(12) **EUROPEAN PATENT APPLICATION**
published in accordance with Art. 153(4) EPC

(43) Date of publication:
**23.09.2026 Bulletin 2026/39**

(21) Application number: **24891255.2**

(22) Date of filing: **01.11.2024**

(51) International Patent Classification (IPC):
***A61K 8/73*** *(2006.01)* ***A61Q 19/10*** *(2006.01)*
***C08B 30/20*** *(2006.01)* ***C11D 3/14*** *(2006.01)*
***C11D 3/22*** *(2006.01)*

(52) Cooperative Patent Classification (CPC):
**A61K 8/73; A61Q 19/10; C08B 30/20; C11D 3/14; C11D 3/22**

(86) International application number:
**PCT/JP2024/039063**

(87) International publication number:
**WO 2025/105216 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.11.2023 JP 2023196068**

(71) Applicant: **J-Oil Mills, Inc.**
**Tokyo 104-0044 (JP)**

(72) Inventors:
- **KASAHARA Tsukasa**
  **Tokyo 104-0044 (JP)**
- **HASHIMOTO Kazuki**
  **Tokyo 104-0044 (JP)**

(74) Representative: **Hoffmann Eitle**
**Patent- und Rechtsanwälte PartmbB**
**Arabellastraße 30**
**81925 München (DE)**

(54) **POWDER**

(57) A powder for a scrubbing agent, comprising: a starch in an amount of 60% by mass or more, and a difference between a heated soluble fraction amount after heating at 80°C for 30 minutes and a cold-water-soluble fraction amount at 25°C is less than 10%.



Processed by Luminess, 75001 PARIS (FR)

## Description

TECHNICAL FIELD

The present invention relates to a powder.

BACKGROUND ART

As a technology related to a material containing starch, there is a technology described in Patent Document 1 (Japanese Unexamined Patent Publication No. 2019-942777). In Patent Document 1, as a technology for providing a scrubbing agent having excellent scrubbing sensation and having low irritation, blending stability in a washing agent, and easy manufacturability (paragraph 0006), a particulate scrubbing agent containing a gelatinized starch and having a gelatinization degree of 10% to 90% is described (Claim 1).

RELATED DOCUMENT

PATENT DOCUMENT

Patent Document 1: Japanese Unexamined Patent Publication No. 2019-942777

SUMMARY OF THE INVENTION

TECHNICAL PROBLEM

As a result of studying the technology described in Patent Document 1, the present inventors have found that there is room for improvement in terms of making an afterfeel of the scrubbing agent preferable even in a case where the particles are blended with the scrubbing agent through a heating step.

The present invention provides a new material that can be used in a scrubbing agent.

SOLUTION TO PROBLEM

According to the present invention, there are provided the following powder, a scrubbing agent and a method for manufacturing the same, a washing method, and a massage method.

[1] A powder which is a powder for a scrubbing agent, the powder containing 60% by mass or more of a starch, in which a difference between a heated soluble fraction amount after heating at 80°C for 30 minutes and a cold-water-soluble fraction amount at 25°C is less than 10%.
[2] The powder according to [1], in which an amylose content of a raw starch of the starch is 50% by mass or more.
[3] The powder according to [1] or [2], in which the heated soluble fraction amount is 10% or less.
[4] The powder according to any one of [1] to [3], in which a heat swelling degree at 80°C is 6.0 or less.
[5] The powder according to any one of [1] to [4], in which a cold-water swelling degree at 25°C is 5.0 or less.
[6] The powder according to any one of [1] to [5], in which a median diameter is 10 μm or more and 1000 μm or less.
[7] The powder according to any one of [1] to [6], in which the starch contains a high-amylose corn starch.
[8] The powder according to [7], in which the starch further contains a starch other than the high-amylose corn starch.
[9] The powder according to [8], in which the starch other than the high-amylose corn starch is one or two or more selected from the group consisting of pea starch, potato starch, and corn starch.
[10] The powder according to any one of [1] to [9], in which a gelatinization degree measured by a β-amylase/pullulanase (BAP) method is less than 10%.
[11] The powder according to any one of [1] to [10], which is for cosmetics.
[12] The powder according to any one of [1] to [11], which is for a skin washing agent or for massage.
[13] A scrubbing agent including the powder according to any one of [1] to [12].
[14] The scrubbing agent according to [13], which is a cosmetic.
[15] The scrubbing agent according to [13] or [14], which is a skin washing agent or a massage agent.
[16] A manufacturing method of a scrubbing agent, including a step of blending the powder according to any one of [1] to [12].
[17] A skin washing method including applying the scrubbing agent according to any one of [13] to [15] to a skin; and washing the skin.
[18] A massage method including applying the scrubbing agent according to any one of [13] to [15] to a skin; and

massaging the skin.

Any combination of each of these configurations or any aspect in which the expression of the present invention is converted between method, apparatus, and the like is also valid as an aspect of the present invention.

## ADVANTAGEOUS EFFECTS OF INVENTION

According to the present invention, it is possible to provide a new material that can be used in a scrubbing agent.

## DESCRIPTION OF EMBODIMENTS

Hereinafter, embodiments of the present invention will be described. Unless particularly stated otherwise, the term "to" in a numerical range indicates being equal to or more than a value and equal to or less than another value, both the values at the two ends being included. In addition, in the present embodiment, a composition can include each component alone or in combination of two or more components.

(Powder)

In the present embodiment, the powder is a powder for a scrubbing agent, containing 60% by mass or more of a starch. In addition, a difference between a heated soluble fraction amount after heating at 80°C for 30 minutes and a cold-water-soluble fraction amount at 25°C of the powder is less than 10%.

Specifically, the powder is a composition containing 60% by mass or more of a starch.

The powder in the present embodiment mainly contains a starch, and a difference between a heated soluble fraction amount and a cold-water-soluble fraction amount is in a specific range. Therefore, the powder can be suitably used in a scrubbing agent. In addition, even in a case where the powder is used in a scrubbing agent obtained through a heating step, an afterfeel of the obtained scrubbing agent can be made suitable. More specifically, by using the powder of the present embodiment, for example, it is also possible to obtain a scrubbing agent having a preferable hardness of granules during washing or massaging and reduced irritation.

In addition, by using the powder of the present embodiment, for example, it is also possible to obtain a scrubbing agent having sufficient detergency and excellent clean feeling after washing. In addition, for example, it is also possible to obtain a scrubbing agent having a reduced irritation to the skin while feeling a massage effect.

Hereinafter, first, components contained in the powder will be described.

The powder contains 60% by mass or more of a starch. The powder may be composed of a starch, or may further contain a component other than the starch.

From the viewpoint of making the hardness of the powder in a case of being used in a scrubbing agent preferable, a content of the starch in the powder is 60% by mass or more, preferably 70% by mass or more, more preferably 80% by mass or more, and still more preferably 90% by mass or more with respect to the entire powder. In addition, the content of the starch in the powder is 100% by mass or less with respect to the entire powder, and may be, for example, 95% by mass or less or 90% by mass or less.

Specific examples of the raw starch blended in the powder include corn-derived starch such as corn starch (regular corn starch), waxy corn starch, and high-amylose corn starch; potato starch; tapioca starch (starch derived from cassava); wheat starch; rice starch; sago starch; sweet potato starch; bean starch such as mung bean starch and pea starch; and processed starch obtained by chemically, physically, or enzymatically processing these.

Examples of the treatment in the processed starch include one or two or more treatments selected from the group consisting of chemical treatments such as an acid treatment, an alkali treatment, an oxidation treatment, an esterification (for example, acetylation) treatment, an etherification (for example, hydroxypropylation) treatment, and a crosslinking treatment; physical treatments such as a heating treatment, an $\alpha$-treatment, a moist heat treatment, a ball milling treatment, and a micro pulverization treatment; and an enzyme treatment.

In addition, from the viewpoint of improving the heat resistance of the powder, the amylose content of the raw starch of the starch is preferably 50% by mass or more, more preferably 55% by mass or more, still more preferably 65% by mass or more, and even more preferably 70% by mass or more.

In addition, the amylose content of the raw starch of the starch is specifically less than 100% by mass, and from the viewpoint of reducing the irritation of the scrubbing agent, it is preferably 95% by mass or less, more preferably 90% by mass or less, and still more preferably 85% by mass or less.

From the viewpoint of improving the heat resistance of the powder, the starch preferably includes one or two or more selected from the group consisting of high-amylose corn starch, corn starch, pea starch, and potato starch, and more preferably includes high-amylose corn starch.

From the viewpoint of improving the heat resistance of the powder, the content of the high-amylose corn starch in

the starch is preferably more than 50% by mass, more preferably 55% by mass or more, still more preferably 60% by mass or more, and even more preferably 75% by mass or more with respect to the entire starch, and may be 100% by mass.

In addition, the content of the high-amylose corn starch in the starch is 100% by mass or less with respect to the entire starch.

From the viewpoint of improving the heat resistance of the powder, it is also preferable that the starch includes high-amylose corn starch and further includes a starch other than the high-amylose corn starch. From the same viewpoint, the starch other than the high-amylose corn starch is preferably one or two or more selected from the group consisting of tapioca starch, rice starch, sweet potato starch, pea starch, potato starch, and corn starch, and more preferably one or two or more selected from the group consisting of pea starch, potato starch, and corn starch.

In this case, the content of the starch other than the high-amylose corn starch in the starch can be, for example, the remainder obtained by excluding the high-amylose corn starch from the starch. More specifically, from the viewpoint of improving the heat resistance of the powder, the content of the starch other than the high-amylose corn starch in the starch is, for example, less than 50% by mass, more preferably 45% by mass or less, still more preferably 40% by mass or less, and even more preferably 25% by mass or less with respect to the entire starch, and may be, for example, 5% by mass or more or 10% by mass or more.

In addition, in a case where the powder contains a component other than the starch, specific examples of the component include amylose such as purified amylose and synthetic amylose; plasticizers such as sugars, glycerin, and sugar alcohols; and water-soluble polymers such as dextrin and polysaccharides.

In a case where the powder contains amylose, from the viewpoint of making the hardness of the powder preferable, the total content of the starch and the amylose in the powder is preferably more than 80% by mass, more preferably 90% by mass or more, still more preferably 95% by mass or more, and even more preferably 97% by mass or more with respect to the entire powder. In addition, the total content of the starch and the amylose in the powder is 100% by mass or less with respect to the entire powder, and may be, for example, 99% by mass or less or 98% by mass or less.

Next, characteristics of the powder will be described.

From the viewpoint of improving the heat resistance of the powder, a difference (hereinafter, also simply referred to as a "soluble fraction amount difference") between a heated soluble fraction amount (hereinafter, also simply referred to as a "heated soluble fraction amount") of the powder after heating at 80°C for 30 minutes and a cold-water-soluble fraction amount (hereinafter, also simply referred to as a "cold-water-soluble fraction amount") of the powder at 25°C is less than 10%, and is preferably 9% or less, more preferably 8% or less, still more preferably 7% or less, and even more preferably 6% or less.

In addition, the soluble fraction amount difference of the powder is specifically 0.1% or more, and may be, for example, 1% or more or 2% or more.

From the viewpoint of improving the heat resistance of the powder, the heated soluble fraction amount of the powder is preferably 10% or less, more preferably 9.5% or less, still more preferably 9% or less, even more preferably 8% or less, and even more preferably 6% or less.

In addition, the heated soluble fraction amount of the powder is specifically 0% or more, and may be, for example, 1% or more or 2% or more.

From the viewpoint of improving the heat resistance of the powder, the cold-water-soluble fraction amount of the powder may be, for example, 5% or less, and is preferably 3% or less, more preferably 2% or less, still more preferably 1% or less, and even more preferably 0.5% or less.

In addition, the cold-water-soluble fraction amount of the powder is specifically 0% or more, and may be, for example, 0.1% or more or 0.2% or more.

From the viewpoint of imparting an appropriate hardness to the powder, a heat swelling degree of the powder at 80°C (hereinafter, also simply referred to as a "heat swelling degree") is preferably 6.0 or less, more preferably 5.5 or less, still more preferably 5.0 or less, even more preferably 4.5 or less, and even more preferably 4.0 or less.

In addition, from the viewpoint of reducing the irritation to the skin, the heat swelling degree is preferably 1.0 or more, more preferably 1.5 or more, and still more preferably 2.0 or more.

From the viewpoint of imparting an appropriate hardness to the powder, a cold-water swelling degree at 25°C (hereinafter, also simply referred to as a "cold-water swelling degree") is preferably 5.0 or less, more preferably 4.5 or less, and still more preferably 4.0 or less.

In addition, from the viewpoint of reducing the irritation to the skin, the cold-water swelling degree is preferably 1.0 or more, more preferably 1.5 or more, and still more preferably 2.0 or more.

The heated soluble fraction amount, the cold-water-soluble fraction amount, and the soluble fraction amount difference are measured by the following method. Hereinafter, the measurement methods of the cold-water swelling degree and the heat swelling degree will be described together.

(Measurement Method 1: Measurement Method of Cold-Water-Soluble Fraction Amount)

1. The sample is heated and dried at 125°C using a moisture meter (for example, MX-50, manufactured by A&D Company, Limited) to measure moisture, and a dry substance mass is calculated from the obtained moisture value.
2. In a state where 1 g of the sample (A) in terms of the mass of dry matter is dispersed in 50 mL of water at 25°C, stirring is gently performed in a constant-temperature tank at 25°C for 30 minutes, and thereafter centrifugation is performed at 3000 rpm for 10 minutes (for example, centrifuge: manufactured by Hitachi Koki Co., Ltd., Hitachi table-top centrifuge CT6E type; rotor: T4SS type swing rotor; adapter: 50TC × 2S adapter) to separate into a precipitated layer and a supernatant layer.
3. The supernatant layer is removed to measure the mass of the precipitated layer, which is defined as $B_{25}$ (g).
4. The mass of the precipitated layer when dried (105°C, constant weight) is measured, and is defined as $C_{25}$ (g).
5. The cold-water-soluble fraction amount and the cold-water swelling degree are measured from the following expression.

$$\text{Cold-water-soluble fraction amount (\%)} = 100 \times (A - C_{25})/A$$

$$\text{Cold water swelling degree} = B_{25}/C_{25}$$

(Measurement Method 2: Measurement Method of Heated soluble fraction amount, Soluble Fraction Amount Difference, and Heat swelling degree)

In the procedure 2 of the measurement method 1, the sample is gently stirred in a constant temperature tank at 80°C for 30 minutes, and then allowed to cool to 30°C, instead of being gently stirred in a constant temperature tank at 25°C for 30 minutes, and then the procedures 1 to 4 are performed according to the measurement method 1 to obtain a precipitated layer mass $B_{80}$ (g) and a dry substance mass $C_{80}$ (g) at 80°C.
Then, the heated soluble fraction amount, the soluble fraction amount difference, and the heat swelling degree are measured from the following expression.

$$\text{Heated soluble fraction amount (\%)} = 100 \times (A - C_{80})/A$$

Soluble fraction amount difference (%) = heated soluble fraction amount - cold-water-soluble fraction amount

$$\text{Heat swelling degree} = B_{80}/C_{80}$$

From the viewpoint of improving the massage property of the scrubbing agent or the detergency in a case of being used in a washing agent, a median diameter of the powder is preferably 10 μm or more, more preferably 50 μm or more, still more preferably 100 μm or more, and even more preferably 120 μm or more.
In addition, from the viewpoint of reducing the irritation to the skin, the particle diameter of the powder is preferably 1,000 μm or less, more preferably 800 μm or less, still more preferably 600 μm or less, even more preferably 400 μm or less, and even more preferably 300 μm or less.
Here, the median diameter of the powder is specifically a median diameter of the powder in water, and more specifically, a median diameter of the powder in water measured at 25°C using a laser diffraction/scattering type particle diameter distribution measuring device.
From the viewpoint of improving the heat resistance of the powder, the gelatinization degree (α degree) measured by the β-amylase/pullulanase (BAP) method is preferably less than 10%, more preferably 9.0% or less, and still more preferably 8.0% or less.
In addition, the gelatinization degree is specifically 0% or more, and may be, for example, 1.0% or more.
Here, the gelatinization degree of the powder can be measured according to the method described in "New measurement method of gelatinization degree and aging degree of starch using β-amylase-pullulanase (BAP) system" on pp. 235 to 240 of Starch Science Vol. 28, No. 4 (1981).

(Production Method)

Next, a manufacturing method of the powder will be described.

The manufacturing method of the powder includes, for example, preparing a raw material component containing a raw starch to obtain a powdery composition. More specifically, the manufacturing method of the powder includes the following steps 1 and 2.

(Step 1) Step of preparing raw material component containing raw starch

(Step 2) Step of granulating raw material component

Here, in order to obtain the powder having the soluble fraction amount difference in the above-described range, it is important to appropriately select the raw material component and the blending of the powder and appropriately select the granulation conditions. More specifically, by appropriately selecting an appropriate type of the raw starch and adjusting the processing conditions (temperature, water addition rate, and the like) to an appropriate range, the powder having the soluble fraction amount difference in a predetermined range can be stably obtained.

In Step 1, the above-described raw material component is prepared as the raw material component.

In addition, as Step 2, a method generally used for granulation can be used, and examples thereof include extrusion granulation, stirring granulation, rolling granulation, dry granulation, and melt granulation, and as an example, a device such as an extruder, a spray dryer, and a mixer can be used.

In addition, Step 2 preferably includes granulating the raw material component by heating and pressurizing the raw material component, more preferably includes granulating the raw material component by heating and pressurizing the raw material component with an extruder, and still more preferably includes granulating the raw material component by heating and pressurizing the raw material component with an extruder under a condition where the gelatinization degree of the starch is less than 10%.

In a case of performing extruder treatment, usually, water is added to the raw material to add moisture content of about 10% to 50% by mass, and then the raw material is heated and expanded, for example, under conditions of a barrel temperature of 30°C to 200°C, an outlet temperature of 100°C to 160°C, and a screw rotation speed of 100 to 1,000 rpm. The water addition rate is adjusted, for example, depending on the type of the raw starch.

Suitable conditions for each condition in the extruder treatment are as follows.

Moisture content (water addition rate to raw material): preferably 12% to 40% by mass, more preferably 15% to 35% by mass, and still more preferably 17% to 32% by mass.

Barrel temperature: preferably 30°C to 190°C, more preferably 30°C to 180°C, and still more preferably 30°C to 170°C.

Outlet temperature: preferably 100°C to 160°C, more preferably 110°C to 150°C, and still more preferably 120°C to 140°C.

Screw rotation speed: preferably 120 to 1,000 rpm, more preferably 140 to 1,000 rpm, and still more preferably 150 to 1,000 rpm.

The granulated product obtained in Step 2 may be used as it is as the powder, or may be crushed as necessary, sieved, and appropriately adjusted in size to be used as the powder.

For example, the particle size of the powder may be adjusted to a sieve underflow fraction of a sieve having an opening of 0.25 mm (60 mesh) in the JIS-Z8801-1 standard, that is, 0.25 mm or less. In addition, a proportion of particles having a dry particle diameter of 0.25 mm or less in the powder may be, for example, 100% by mass or less or 95% by mass or less.

In addition, for example, the particle size of the powder may be adjusted to a sieve underflow fraction of a sieve having an opening of 0.15 mm (100 mesh) in the JIS-Z8801-1 standard, that is, 0.15 mm or less. In addition, a proportion of particles having a dry particle diameter of 0.15 mm or less in the powder may be, for example, 100% by mass or less or 95% by mass or less.

The powder obtained in the present embodiment can be used as a scrubbing agent as it is or in combination with other components.

From the viewpoint of reducing the irritation to the body such as the skin, the powder is preferably for cosmetics.

In addition, from the viewpoint of obtaining a preferable scrubbing effect, the powder is preferably for a washing agent such as a skin washing agent or for massage.

(Scrubbing Agent)

In the present embodiment, the scrubbing agent specifically includes the powder in the present embodiment. The scrubbing agent may be composed of the powder, or may be further blended with a component other than the powder.

Specifically, the scrubbing agent is a formulation for personal care such as a washing agent and a massage agent, and is preferably a skin washing agent or a massage agent. In addition, the scrubbing agent is preferably a cosmetic. The cosmetics will be described later.

In addition, the scrubbing agent may be applied to animals (for example, mammals) other than humans, cosmetics, and the like.

Specific examples of the washing agent include a washing agent for personal care. Examples of the washing agent for personal care include a skin washing agent such as a makeup remover, a facial cleanser, a hand soap, a body soap, and a skin washing powder; a hair washing agent such as a shampoo and a hair washing powder; and an oral washing agent such as a toothpaste and a mouthwash. Since the washing agent has excellent low irritation to the skin, the washing agent is preferably a skin washing agent.

In addition, specific examples of the massage agent include a massage agent for personal care such as a skin massage agent, a scalp massage agent, and a nail scrub.

Examples of the formulation of the scrubbing agent include a liquid such as an aqueous solution, an emulsion, and a suspension; a semisolid such as a gel and a cream; and a solid such as a powder, a granule, and a solid.

From the viewpoint of improving the washing or massage effect and the afterfeel, a content of the powder in the scrubbing agent is preferably 0.1% by mass or more, more preferably 1% by mass or more, still more preferably 2% by mass or more, even more preferably 3% by mass or more, and even more preferably 5% by mass or more with respect to the entire scrubbing agent.

In addition, the content of the powder in the scrubbing agent is 100% by mass or less. In addition, an upper limit of the content of the powder may be appropriately set depending on the type or the formulation of the scrubbing agent, and may be, for example, 20% by mass or less or 10% by mass or less with respect to the entire scrubbing agent.

In addition, examples of other components contained in the scrubbing agent include components contained in the cosmetics described later. The other components can be selected depending on the type or the formulation of the scrubbing agent.

The manufacturing method of the scrubbing agent includes, for example, a step of blending the powder in the present embodiment. More specifically, the manufacturing method of the scrubbing agent can be obtained by adding the powder in the present embodiment to an intermediate product or a final product in a manufacturing step of the scrubbing agent. In addition, the manufacturing method of the scrubbing agent can be selected depending on the type or the formulation of the scrubbing agent, and the powder in the present embodiment can be blended in the manufacturing step.

In the present embodiment, the washing method includes applying the scrubbing agent in the present embodiment to a subject and washing the subject. In addition, the skin washing method includes, for example, applying the scrubbing agent in the present embodiment to the skin and washing the skin, and preferably includes applying the scrubbing agent in the present embodiment to the skin. In addition, the skin may be massaged after applying the washing agent to the skin.

In the present embodiment, the massage method includes applying the scrubbing agent in the present embodiment to a subject and massaging the subject. In addition, the skin massage method includes, for example, applying the scrubbing agent in the present embodiment to the skin and massaging the skin, and preferably includes applying the scrubbing agent to the skin and massaging the skin.

(Cosmetic)

In the present embodiment, the cosmetic includes the scrubbing agent in the present embodiment. The cosmetic may be composed of the scrubbing agent, or may be further blended with a component other than the scrubbing agent.

The cosmetic can be used not only for humans but also for pets such as dogs and cats and for articles such as cosmetic brushes, and is preferably used for humans.

The cosmetic may be any cosmetic that can be blended with the scrubbing agent in the present embodiment, and specific examples thereof include the various washing agents and massage agents described above, and also include cosmetics for skin such as a facial toner, an emulsion, a beauty liquid, a moisturizing cream, a pack, a massage or a cold cream, a skin care cream, a cosmetic oil, a hand cream or a lotion, and a body cream or a lotion, and a baby lotion; cosmetics for hair such as a rinse, a hair treatment, a hair cream, a hair mousse, a hair wax, a hair spray, and a hair growth agent; oral cosmetics; cosmetics for nails such as a manicure; cosmetics for beard such as a shaving cream or a foam; deodorant cosmetics such as an antiperspirant; and other special-purpose cosmetics such as a sunscreen and a suntan cosmetic.

The cosmetic is preferably one selected from the group consisting of a cosmetic for skin, a finishing cosmetic, and a cosmetic for hair, and more preferably one selected from the group consisting of a cosmetic for skin and a finishing cosmetic. Here, the cosmetic also includes a non-pharmaceutical preparation.

The formulation of the cosmetic is not limited. The formulation may be any of a liquid such as an aqueous solution, an emulsion, and a suspension; a semisolid formulation such as a gel and a cream; or a solid formulation such as a powder,

a granule, and a solid. In addition, the formulation may be an emulsion formulation such as a cream and an emulsion, an oil gel formulation such as a lipstick, a powder formulation such as a foundation, and an aerosol formulation such as a hair styling agent.

From the viewpoint of improving the afterfeel of the cosmetic, a content of the scrubbing agent in the cosmetic is preferably 0.1% by mass or more, more preferably 1% by mass or more, still more preferably 2% by mass or more, even more preferably 3% by mass or more, and even more preferably 5% by mass or more with respect to the entire cosmetic.

In addition, the content of the scrubbing agent in the cosmetic is 100% by mass or less. In addition, an upper limit of the content of the powder may be appropriately set depending on the type or the formulation of the cosmetic, and may be, for example, 20% by mass or less or 10% by mass or less with respect to the entire cosmetic.

Examples of the component other than the powder blended in the cosmetic include a component commonly used in cosmetics, for example, an oily component, an aqueous component, a whitening agent, a moisturizer, a stabilizer, an antioxidant, a preservative, a pH adjuster, an ultraviolet absorber, a thickener, a powder component, a coloring material, a fragrance, a surfactant, a nutritional component for the skin, and a beauty care ingredient.

Among these, examples of the oily component include one or two or more selected from the group consisting of an oil, a hydrocarbon, a higher fatty acid, a higher alcohol, a wax, an ester oil, and a silicone oil.

Examples of the oil and fat include plant oils such as soybean oil, rapeseed oil, palm oil, corn oil, olive oil, sesame oil, perilla oil, linseed oil, safflower oil, sunflower oil, cottonseed oil, rice oil, peanut oil, cocoa butter, palm kernel oil, coconut oil, camellia oil, tea oil, mustard oil, kapok oil, kaya oil, walnut oil, poppy oil, macadamia nut oil, avocado oil, Persian oil, shea butter, and rosehip oil; animal oils such as beef tallow, pork fat, milk fat, chicken oil, fish oil, horse fat, mink oil, turtle oil, and egg yolk oil; and synthetic oils such as medium-chain fatty acid triglyceride. In addition, processed oils obtained by subjecting these oils to separation, hydrogenation, ester exchange, and the like are also included.

Examples of the hydrocarbon include one or two or more selected from the group consisting of liquid paraffin, vaseline, paraffin wax, and squalane.

Examples of the higher fatty acid include lauric acid, myristic acid, palmitic acid, stearic acid, behenic acid, oleic acid, isostearic acid, linoleic acid, linolenic acid, eicosapentaenoic acid, and docosahexaenoic acid.

Examples of the higher alcohol include lauryl alcohol, cetanol, and stearyl alcohol.

Examples of the wax include beeswax, carnauba wax, rice wax, lanolin, and jojoba oil.

Examples of the ester oil include isopropyl myristate, isopropyl palmitate, cetyl ethylhexanoate, triethylhexanoate, and butyl oleate.

Examples of the silicone oil include dimethicone, cyclopentasiloxane, and methylphenylsiloxane.

Examples of the aqueous component include water; ethanol; glycols such as propylene glycol, 1,3-butylene glycol, dipropylene glycol, tripropylene glycol, 1,2-pentanediol, and polyethylene glycol; glycerols such as glycerin, diglycerin, and polyglycerin; and one or two or more selected from the group consisting of plant extracts such as aloe vera, witch hazel, hamamelis, cucumber, lemon, lavender, and rose.

In a case where the cosmetic contains an oily component, the oily component may be blended depending on the type of the cosmetic, and the blending amount thereof is not limited, but is preferably 0.1 parts by mass or more and 100 parts by mass or less, more preferably 0.4 parts by mass or more and 40 parts by mass or less, and still more preferably 0.6 parts by mass or more and 10 parts by mass or less with respect to 1 part by mass of the powder.

In a case where the cosmetic contains an oily component and water, the oily component and the water may be blended depending on the type of the cosmetic, and the blending amounts thereof are not limited, but are preferably 0.1 parts by mass or more and 90 parts by mass or less of the oily component and 0.1 parts by mass or more and 90 parts by mass or less of the water with respect to 1 part by mass of the powder, more preferably 0.4 parts by mass or more and 40 parts by mass or less of the oily component and 2 parts by mass or more and 40 parts by mass or less of the water with respect to 1 part by mass of the powder, and still more preferably 0.6 parts by mass or more and 10 parts by mass or less of the oily component and 3 parts by mass or more and 10 parts by mass or less of the water with respect to 1 part by mass of the powder.

The cosmetic can be manufactured, for example, by adding the powder to an intermediate product or a final product in a manufacturing step of the cosmetic.

Specific examples of the manufacturing method of the cosmetic include a manufacturing method of a cosmetic by adding and mixing the powder in the present embodiment to other cosmetic components; and a manufacturing method of a cosmetic by adding and mixing a composition for a cosmetic obtained by mixing the powder in the present embodiment and other cosmetic components to other cosmetic components.

More specific examples of the manufacturing method of the cosmetic include a manufacturing method of a cosmetic by adding and mixing the powder in the present embodiment to an oily component; a manufacturing method of a cosmetic by adding and mixing the powder in the present embodiment to other powder components; a manufacturing method of a cosmetic by adding and mixing the powder in the present embodiment to an oily component and an aqueous component; a manufacturing method of a cosmetic by adding and mixing a composition for a cosmetic obtained by mixing the powder in the present embodiment and an oily component to other cosmetic components; a manufacturing method of a

cosmetic by adding and mixing a composition for a cosmetic obtained by mixing the powder in the present embodiment and other powder components to other cosmetic components; and a manufacturing method of a cosmetic by adding and mixing a composition for a cosmetic obtained by mixing the powder in the present embodiment, an oily component, and an aqueous component to other cosmetic components.

Although the embodiments of the present invention have been described above, these are examples of the present invention, and various configurations other than the above can be adopted. Examples

Examples of the present invention are shown below, but the scope of the present invention is not limited to these Examples.

The following materials were mainly used as raw materials.

(Starch)

High-amylose waxy corn starch: J-OIL MILLS HS-7, manufactured by J-OIL MILLS Co., Ltd., amylose content: 72% by mass
Pea starch: manufactured by Puris, amylose content: 43% by mass
Regular corn starch: J-OIL MILLS corn starch Y, manufactured by J-OIL MILLS Co., Ltd., amylose content: 26% by mass
Potato starch: GELCARE BP-200, manufactured by J-OIL MILLS Co., Ltd., amylose content: 35% by mass

(Others)

Coffee powder scrub: NIKKOL Cafe scrub UC, manufactured by NIKKO CHEMICAL CO., LTD.

Glucomannnan scrub: BIODIOS Scrub White, manufactured by Shimizu Chemical Co., Ltd.

(Examples 1 to 11, Comparative Examples 1 to 7)

The powder of each example was manufactured using a starch, and the characteristics were measured.

(Manufacturing Method of Powder)

In each of Examples other than Comparative Examples 6 and 7, the powder was manufactured by extrusion granulation using a Process 11 (manufactured by Thermo Scientific) which is a twinscrew extruder.

The extrusion granulation using the extruder was carried out under the following conditions: a barrel temperature of 50°C to 140°C, a die temperature of 110°C to 140°C, a feed amount (in Table 1, "Raw material input amount") of 6.9 to 7.9 g/min, an amount of water added of 22.8% to 33.6% (relative to the powder), a die diameter of 1.5 to 2.0 mm, an LD ratio of 40, and a screw rotation speed of 150 rpm.

More specific manufacturing conditions of each example are shown in Table 1.

In addition, the characteristics of the powder obtained in each example were measured by the following method. The measurement results are shown in Table 2.

(Measurement of Characteristics)

(Cold-Water Swelling Degree, Cold-Water-Soluble Fraction Amount)

1. As a measurement sample, a composition was crushed in advance, and a sample in which the particle size was adjusted to a sieve underflow fraction of a sieve having an opening of 0.25 mm (60 mesh) in the JIS-Z8801-1 standard, that is, 0.25 mm or less was used.
2. The sample was heated and dried at 125°C using a moisture meter (manufactured by A&D Company, Limited, model number MX-50) to measure moisture, and a dry substance mass was calculated from the obtained moisture value.
3. 1 g of the sample (A) in terms of the mass of dry matter was weighed in a 50 mL tube in terms of the dry substance mass.
4. While stirring with a vortex mixer, water was added to the tube up to the 50 mL mark.
5. The mixture was mixed by inversion 5 times to disperse the precipitation, stirred with a vortex mixer for 10 seconds,

and allowed to stand in a constant temperature tank at 25°C for 30 minutes.
6. The mixture was centrifuged (3,000 rpm, 10 min) and separated into a precipitated layer and a supernatant layer. (Centrifuge: Hitachi desktop centrifuge CT6E manufactured by Hitachi Koki Co., Ltd.; rotor: T4SS swing rotor; adapter: 50TCx2S adapter)
7. The supernatant layer was removed with a pipette, and the mass of the precipitated layer was measured and denoted by B25.
8. The mass of the precipitated layer when dried (105°C, 24 h) was measured, and defined as $C_{25}$.
9. The cold-water-soluble fraction amount and the cold-water swelling degree were obtained from the following expression.

$$\text{Cold-water-soluble fraction amount (\%)} = 100 \times (A - C_{25})/A$$

$$\text{Cold water swelling degree} = B_{25}/C_{25}$$

(Heat swelling degree, Soluble Fraction Amount Difference, Heated soluble fraction amount)

1. As a measurement sample, a composition was crushed in advance, and a sample in which the particle size was adjusted to a sieve underflow fraction of a sieve having an opening of 0.25 mm (60 mesh) in the JIS-Z8801-1 standard, that is, 0.25 mm or less was used.
2. The sample was heated and dried at 125°C using a moisture meter (manufactured by A&D Company, Limited, model number MX-50) to measure moisture, and a dry substance mass was calculated from the obtained moisture value.
3. 1 g of the sample (A) in terms of the mass of dry matter was weighed in a 50 mL tube in terms of the dry substance mass.
4. While stirring with a vortex mixer, water was added to the tube up to the 50 mL mark.
5. The mixture was mixed by inversion 5 times to disperse the precipitation, stirred with a vortex mixer for 10 seconds, heated in a constant temperature tank at 80°C for 30 minutes, and then cooled to 30°C as it is.
6. The mixture was centrifuged (3,000 rpm, 10 min) and separated into a precipitated layer and a supernatant layer. (Centrifuge: Hitachi desktop centrifuge CT6E manufactured by Hitachi Koki Co., Ltd.; rotor: T4SS swing rotor; adapter: 50TCx2S adapter)
7. The supernatant was removed with a pipette, and the mass of the precipitated layer was measured and denoted by $B_{80}$.
8. The mass of the precipitated layer when dried (105°C, 24 h) was measured, and defined as $C_{80}$.
9. The heated soluble fraction amount, the soluble fraction amount difference, and the heat swelling degree were measured from the following expression.

$$\text{Heated soluble fraction amount (\%)} = 100 \times (A - C_{80})/A$$

Soluble fraction amount difference (%) = heated soluble fraction amount - cold-water-soluble fraction amount

$$\text{Heat swelling degree} = B_{80}/C_{80}$$

(Median Diameter)

The particle size distribution of the powder of each example at 25°C was measured using a laser diffraction/-scattering type particle diameter distribution measuring device (manufactured by HORIBA, Ltd., model number LA-960v2) with water as a dispersion medium, and the median diameter was obtained based on the volume-based particle size distribution.

($\alpha$-Conversion Degree)

The $\alpha$-conversion degree of the starch constituting the powder was measured by a $\beta$-amylase-pullulanase (BAP)

method.

1. As a measurement sample, a composition was crushed in advance, and a sample in which the particle size was adjusted to a sieve underflow fraction of a sieve having an opening of 0.15 mm (100 mesh) in the JIS-Z8801-1 standard, that is, 0.15 mm or less was used.
2. The α-conversion degree of the starch in the granular substance was measured according to the method described in "New measurement method of gelatinization degree and aging degree of starch using β-amylase-pullulanase (BAP) system" on pp. 235 to 240 of Starch Vol. 28, No. 4 (1981).

(Comparative Example 6)

The commercially available coffee powder scrub was used as the powder of the present example as it is.

(Comparative Example 7)

The commercially available glucomannnan scrub was used as the powder of the present example as it is.

[Table 1]

Table 1

| | Raw material (%:% by mass) | | | | Amylose content of raw starch | Extruder: operating conditions | | | | | | | |
|---|---|---|---|---|---|---|---|---|---|---|---|---|---|
| | High-amylose corn starch | Regular corn starch | Potato starch | Pea starch | | Raw material input amount (g/min) | Screw rotation speed (rpm) | Water addition rate (% to raw material) | Device name | Die diameter | Barrel temperature | Die temperature | Outlet temperature |
| Example 1 | 100% | | | | 72.0% | 7.6 | 150 | 30.6% | Process 11 | Φ1.5 mm | 50°C→120°C | 120 | 118 |
| Example 2 | 100% | | | | 72.0% | 7.6 | 150 | 30.6% | Process 11 | Φ1.5 mm | 50°C→140°C | 140 | 133 |
| Example 3 | 80% | | | 20% | 66.2% | 7.8 | 150 | 30.1% | Process 11 | Φ2.0 mm | 50°C→120°C | 120 | 116 |
| Example 4 | 80% | | | 20% | 66.2% | 7.8 | 150 | 30.1% | Process 11 | Φ2.0 mm | 50°C→140°C | 140 | 136 |
| Example 5 | 80% | | 20% | | 64.6% | 7.8 | 150 | 30.3% | Process 11 | Φ1.5 mm | 50°C→120°C | 120 | 118 |
| Example 6 | 80% | | 20% | | 64.6% | 7.8 | 150 | 30.3% | Process 11 | Φ1.5 mm | 50°C→140°C | 140 | 135 |
| Example 7 | 80% | 20% | | | 62.8% | 7.6 | 150 | 30.2% | Process 11 | Φ1.5 mm | 50°C→120°C | 120 | 117 |
| Example 8 | 80% | 20% | | | 62.8% | 7.6 | 15C | 30.2% | Process 11 | Φ1.5 mm | 50°C→140°C | 140 | 136 |
| Example 9 | 70% | 30% | | | 58.2% | 7.9 | 15C | 30.1% | Process 11 | Φ1.5 mm | 50°C→120°C | 120 | 116 |
| Example 10 | 70% | 30% | | | 58.2% | 7.9 | 15C | 30.1% | Process 11 | Φ1.5 mm | 50°C→140°C | 140 | 136 |
| Example 11 | 60% | 40% | | | 53.6% | 7.6 | 15C | 30.2% | Process 11 | Φ1.5 mm | 50°C→140°C | 140 | 136 |
| Comparative Example 1 | 50% | 50% | | | 49.0% | 7.7 | 15C | 29.8% | Process 11 | Φ1.5 mm | 50°C→120°C | 120 | 117 |

(continued)

| | Raw material (%:% by mass) | | | | Amylose content of raw starch | Extruder: operating conditions | | | | | | | |
|---|---|---|---|---|---|---|---|---|---|---|---|---|---|
| | High-amylose corn starch | Regular corn starch | Potato starch | Pea starch | | Raw material input amount (g/min) | Screw rotation speed (rpm) | Water addition rate (% to raw material) | Device name | Die diameter | Barrel temperature | Die temperature | Outlet temperature |
| Comparative Example 2 | | | | 100% | 43.0% | 7.5 | 15C | 30.8% | Process 11 | Φ1.5 mm | 50°C→120°C | 120 | 117 |
| Comparative Example 3 | 30% | 70% | | | 39.8% | 7.6 | 150 | 30.4% | Process 11 | Φ1.5 mm | 50°C→120°C | 120 | 117 |
| Comparative Example 4 | 30% | 70% | | | 39.8% | 7.6 | 150 | 22. 8% | Process 11 | Φ1.5 mm | 50°C→140°C | 140 | 137 |
| Comparative Example 5 | | 100% | | | 26.0% | 6.9 | 150 | 33.6% | Process 11 | Φ1.5 mm | 50°C→110°C | 110 | 108 |

[Table 2]

Table 2

| | α-Conversion degree | Cold-water swelling degree | Cold-water-soluble fraction amount | Heat swelling degree | Heated soluble fraction amount | Soluble fraction amount difference | Median diameter |
|---|---|---|---|---|---|---|---|
| Example 1 | 2.3% | 3.41 | 0.0% | 3.76 | 3.2% | 3.2% | 132 |
| Example 2 | 7.3% | 3.55 | 0.3% | 3.95 | 5.0% | 4.7% | 143 |
| Example 3 | 7.2% | 3.75 | 0.7% | 4.43 | 5.3% | 4.6% | 151 |
| Example 4 | 6.5% | 3.78 | 0.6% | 4.49 | 7.0% | 6.4% | 148 |
| Example 5 | 2.1% | 3.62 | 0.4% | 5.46 | 7.0% | 6.6% | 143 |
| Example 6 | 4.4% | 3.58 | 1.0% | 4.80 | 7.0% | 6.0% | 140 |
| Example 7 | 3.9% | 3.82 | 0.2% | 4.65 | 4.9% | 4.7% | 155 |
| Example 8 | 5.9% | 3.61 | 0.1% | 4.74 | 7.0% | 6.9% | 143 |
| Example 9 | 5.9% | 4.12 | 0.0% | 5.30 | 5.1% | 5.1% | 142 |
| Example 10 | 5.0% | 3.71 | 0.0% | 5.02 | 5.5% | 5.5% | 135 |
| Example 11 | 5.4% | 4.22 | 3.9% | 5.77 | 9.2% | 5.3% | 153 |
| Comparative Example 1 | 24.0% | 5.48 | 1.7% | 6.18 | 11.7% | 10.0% | 166 |
| Comparative Example 2 | 39.9% | 10.15 | 14.1% | 15.44 | 26.5% | 12.4% | 294 |
| Comparative Example 3 | 19.8% | 6.15 | 1.6% | 7.48 | 13.1% | 11.5% | 166 |
| Comparative Example 4 | 56.4% | 7.21 | 10.5% | 8.29 | 22.0% | 11.5% | 149 |
| Comparative Example 5 | 66.6% | 13.18 | 14.3% | 13.63 | 29.2% | 14.9% | 165 |

(Evaluation)

The following evaluations were performed on the powder obtained in some examples. For Comparative Examples 6 and 7, only the non-heated evaluation was performed among the following evaluations. Table 3 shows the results.

(Evaluation 1) Preparation and Evaluation of Facial Foam with Scrub

1. 5 parts of the powder of each example was added to 95 parts of a commercially available facial foam (manufactured by Kao Corporation, Biore Skin Care Facial Cleanser Moisture), and the mixture was well mixed. This was used as a facial foam with scrub.
2. The facial foam with scrub was divided into a non-heated evaluation and a heated evaluation, and the heated evaluation was heated in a constant temperature tank at 80°C for 30 minutes.
3. 100 parts by mass of water were added to 1 part by mass of each of the facial foam with scrub for non-heated evaluation and the facial foam with scrub for heated evaluation, and the mixture was well mixed to obtain an evaluation sample of each example.
4. The detergency and the after-washing feeling were evaluated by the following procedure.

(Evaluation Method of Detergency and After-Washing Feeling)

1. The lipstick (KANCOLLE MATTE LIP STICK, manufactured by ERATO Co., Ltd.) was applied to the inside of the arm 5 times.
2. One drop of the evaluation sample of each example was applied.
3. The inside of the arm was rubbed with a finger 10 times as if the lipstick was removed. In this case, the finger was not particularly pressed with force.
4. The inside of the arm was rinsed with water for 10 seconds.
5. The detergency (the degree to which the lipstick color was washed off) and the after-washing feeling (the massage effect and the clean feeling of the skin) were evaluated according to the following standards. The evaluation was performed by three persons, and the average was used as the evaluation score. A score of equal to or higher than 3.0 was considered acceptable.

(Detergency)

5: The lipstick was removed by 90% or more
4: The lipstick was removed by about 70%
3: The lipstick was removed by about 50%
2: The lipstick was removed by about 30%
1: The lipstick was removed by 10% or less

(After-Washing Feeling)

5: The massage effect during washing is sufficient, and a very clean feeling is felt
4: The massage effect during washing is felt, and a clean feeling is felt
3: The massage effect during washing is slightly felt, and a slightly clean feeling is felt
2: The massage effect during washing is almost non-existent, and a clean feeling is not felt much
1: The massage effect during washing is not felt at all, and a clean feeling is not felt at all

(Evaluation 2) Preparation and Evaluation of Scrub Water Suspension

1. 95 parts by mass of water and 5 parts by mass of the powder of each example were well mixed in a ratio to obtain a scrub water suspension.
2. The scrub water suspension was divided into a non-heated evaluation and a heated evaluation, and the heated evaluation was heated in a hot water bath at 80°C for 30 minutes.
3. The scrub water suspension for non-heated evaluation and the scrub water suspension for heated evaluation were used as evaluation samples as they are.
4. The hardness of the granules and the irritation were evaluated by the following procedure.

(Evaluation Method of Hardness of Granules and Non-Irritation)

1. An appropriate amount of the evaluation sample of each example was dropped onto the back of the hand.
2. The hardness of the granules (hardness felt when the granules are pressed with a finger) and the non-irritation (lack of irritation such as pain) were evaluated by pressing or rubbing the particles that were dropped with a finger.

The evaluation was performed by three persons, and the average was used as the evaluation score. 3.0 points or more was considered to be a pass for the hardness of the granules, and 1.0 point or more was considered to be a pass for the non-irritation.

(Hardness of Granules)

5: Very hard, and a strong granule feeling is felt

4: Somewhat hard, and a granule feeling is felt

3: Somewhat hard, and a granule feeling is slightly felt

2: Soft, and a granule feeling is weak

1: Very soft, and a granule feeling is very weak

0: The granules are easily broken, and there is no granule feeling at all

(Non-Irritation)

2: No irritation

1: Almost no irritation

0: Irritation is too strong

[Table 3]

Table 3

| | Detergency | | After-washing feeling | | Hardness of granules | | Non-irritation | |
|---|---|---|---|---|---|---|---|---|
| | Non-heated | Heated | Non-heated | Heated | Non-heated | Heated | Non-heated | Heated |
| Example 1 | 4.7 | 4.0 | 5.0 | 4.0 | 5.0 | 5.0 | 1 | 1 |
| Example 3 | 4.7 | 4.0 | 4.7 | 4.0 | 4.7 | 4.0 | 1 | 1 |
| Example 5 | 4.0 | 3.7 | 4.0 | 4.0 | 4.0 | 3.3 | 1 | 2 |
| Example 7 | 3.7 | 3.3 | 3.3 | 3.0 | 4.0 | 3.3 | 1 | 2 |
| Example 9 | 4.0 | 3.3 | 3.7 | 3.3 | 3.3 | 3.0 | 2 | 2 |
| Example 11 | 3.3 | 3.3 | 3.3 | 3.3 | 3.3 | 3.0 | 2 | 2 |
| Comparative Example 1 | 3.0 | 3.0 | 3.0 | 3.0 | 2.3 | 1.0 | 2 | 2 |
| Comparative Example 2 | 2.3 | 2.3 | 2.3 | 1.7 | 1.0 | 0.7 | 2 | 2 |
| Comparative Example 3 | 1.7 | 1.0 | 1.0 | 1.0 | 2.0 | 0.0 | 2 | 2 |
| Comparative Example 5 | 1.0 | 1.0 | 1.0 | 1.0 | 1.0 | 0.0 | 2 | 2 |
| Comparative Example 6 | 3.0 | - | 2.7 | - | 5.0 | - | 0 | - |
| Comparative Example 7 | 1.7 | - | 1.3 | - | 3.7 | - | 1 | - |

From Table 3, the powder obtained in each of Examples exhibited an excellent balance of each of the effects of detergency, after-washing feeling, hardness of granules, and irritation in both the non-heated evaluation and the heated evaluation. In addition, by using the powder obtained in each of Examples, a preferable massage effect on the skin was felt.

This application claims priority based on Japanese Patent Application No. 2023-196068 filed on November 17, 2023, the entire disclosure of which is incorporated herein.

## Claims

**1.** A powder which is a powder for a scrubbing agent, the powder containing 60% by mass or more of a starch, wherein a difference between a heated soluble fraction amount after heating at 80°C for 30 minutes and a cold-water-soluble fraction amount at 25°C is less than 10%.

**2.** The powder according to claim 1,
wherein an amylose content of a raw starch of the starch is 50% by mass or more.

**3.** The powder according to claim 1,
wherein the heated soluble fraction amount is 10% or less.

**4.** The powder according to claim 1,
wherein a heat swelling degree at 80°C is 6.0 or less.

**5.** The powder according to claim 1,
wherein a cold-water swelling degree at 25°C is 5.0 or less.

**6.** The powder according to claim 1,
wherein a median diameter is 10 μm or more and 1000 μm or less.

**7.** The powder according to claim 1,
wherein the starch contains a high-amylose corn starch.

**8.** The powder according to claim 7,
wherein the starch further contains a starch other than the high-amylose corn starch.

**9.** The powder according to claim 8,
wherein the starch other than the high-amylose corn starch is one or two or more selected from the group consisting of pea starch, potato starch, and corn starch.

**10.** The powder according to claim 1,
wherein a gelatinization degree measured by a β-amylase/pullulanase (BAP) method is less than 10%.

**11.** The powder according to claim 1, which is for cosmetics.

**12.** The powder according to claim 1, which is for a skin washing agent or for massage.

**13.** A scrubbing agent containing the powder according to any one of claims 1 to 12.

**14.** The scrubbing agent according to claim 13, which is a cosmetic.

**15.** The scrubbing agent according to claim 14, which is a skin washing agent or a massage agent.

**16.** A manufacturing method of a scrubbing agent, comprising:
a step of blending the powder according to any one of claims 1 to 12.

**17.** A skin washing method comprising:

applying the scrubbing agent according to claim 13 to a skin; and
washing the skin.

**18.** A massage method comprising:

applying the scrubbing agent according to claim 13 to a skin; and
massaging the skin.

**INTERNATIONAL SEARCH REPORT**

International application No.
**PCT/JP2024/039063**

**A. CLASSIFICATION OF SUBJECT MATTER**

***A61K 8/73***(2006.01)i; ***A61Q 19/10***(2006.01)i; ***C08B 30/20***(2006.01)i; ***C11D 3/14***(2006.01)i; ***C11D 3/22***(2006.01)i
FI: A61K8/73; A61Q19/10; C08B30/20; C11D3/14; C11D3/22

According to International Patent Classification (IPC) or to both national classification and IPC

**B. FIELDS SEARCHED**

Minimum documentation searched (classification system followed by classification symbols)

A61K8/73; A61Q19/10; C08B30/20; C11D3/14; C11D3/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2019-94277 A (NICHIDENKAGAKU K.K) 20 June 2019 (2019-06-20) claim 1, paragraphs [0007], [0011], [0014], [0023], [0033]-[0034], [0036], table 1 | 1-9, 11-18 |
| A | claim 1, paragraphs [0007], [0011], [0014], [0023], [0033]-[0034], [0036], table 1 | 10 |
| A | WO 2023/008416 A1 (J-OIL MILLS, INC.) 02 February 2023 (2023-02-02) entire text | 1-18 |
| A | JP 2023-118627 A (UNIV. YAMAGATA) 25 August 2023 (2023-08-25) entire text, all drawings | 1-18 |
| A | JP 2022-178928 A (NATIONAL AGRICULTURE AND FOOD RESEARCH ORGANIZATION) 02 December 2022 (2022-12-02) entire text, all drawings | 1-18 |
| A | JP 8-9907 A (HONEN CORP.) 16 January 1996 (1996-01-16) entire text, all drawings | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 December 2024** | **14 January 2025** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | Telephone No. |

Form PCT/ISA/210 (second sheet) (July 2022)

INTERNATIONAL SEARCH REPORT

International application No.
**PCT/JP2024/039063**

**C. DOCUMENTS CONSIDERED TO BE RELEVANT**

| Category* | Citation of document, with indication, where appropriate, of the relevant passages | Relevant to claim No. |
|---|---|---|
| A | WO 2020/090994 A1 (SANWA STARCH CO., LTD.) 07 May 2020 (2020-05-07) entire text, all drawings | 1-18 |

Form PCT/ISA/210 (second sheet) (July 2022)

**INTERNATIONAL SEARCH REPORT**
**Information on patent family members**

International application No.
**PCT/JP2024/039063**

| Patent document cited in search report | Publication date (day/month/year) | Patent family member(s) | Publication date (day/month/year) |
|---|---|---|---|
| JP 2019-94277 A | 20 June 2019 | WO 2019/098358 A1 | |
| WO 2023/008416 A1 | 02 February 2023 | (Family: none) | |
| JP 2023-118627 A | 25 August 2023 | (Family: none) | |
| JP 2022-178928 A | 02 December 2022 | (Family: none) | |
| JP 8-9907 A | 16 January 1996 | (Family: none) | |
| WO 2020/090994 A1 | 07 May 2020 | US 2022/0002443 A1<br>entire text, all drawings<br>EP 3874957 A1<br>CN 112996394 A<br>JP 2022-137180 A<br>JP 2022-9099 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019942777 A
- JP 2023196068 A


### Non-patent literature cited in the description


- New measurement method of gelatinization degree and aging degree of starch using β-amylase-pullulanase (BAP) system. *Starch Science*, 1981, vol. 28 (4), 235-240
- New measurement method of gelatinization degree and aging degree of starch using β-amylase-pullulanase (BAP) system. *Starch*, 1981, vol. 28 (4), 235-240